# EUROPEAN PATENT SPECIFICATION

(11) **EP 0 788 811 B1**
(45) Date of publication and mention of the grant of the patent: **20.08.2003**
(21) Application number: 97200303.2
(22) Date of filing: 04.02.1997
(51) Int. Cl.: A61N 1/32

(54) **A device for application of endermic electrotherapeutic treatments to a human body**
Vorrichtung zur Behandlung des menschlichen Körpers durch endermatische Elektrotherapie
Appareil pour appliquer au corps humain un traitement endermique d'électrothérapie

(30) Priority: 07.02.1996 IT MI960217
(43) Date of publication of application: 13.08.1997
(73) Proprietor: ZOT NEDERLAND B.V., 3735 KA Bosch en Duin (NL)
(72) Inventor: Maida, Massimo Maurizio, 54037 Marina di Massa (Massa Carrara) (IT)
(74) Representative: Faraggiana, Vittorio, Dr. Ing.

(56) References cited:
- EP-A- 0 128 103
- EP-A- 0 193 480
- FR-A- 1 541 165
- FR-A- 2 502 015
- LU-A- 60 281
- US-A- 2 842 135

## Description

The present invention relates to a device for application of electrotherapeutic treatments, in particular for treating pathologies such as cellulitis.

For curing a patient of these pathologies, several different treatments are now employed, which differ from each other based on the utilized operation principles.

One of these treatments is represented by electrolipolysis. It consists in creating a localized electric field, generally by driving needle-shaped electrodes into the skin. Passage of electric current through the cellulitis mass causes a local increase in the production of substances capable of promoting removal of fats from the inside of the cells.

Another treatment consists of iontophoresis utilizing the migration principle of ions from within an electric field of weak-intensity current to cause penetration of drugs and active ingredients until the deepest layers of the skin through capillaries, sweat glands and hair follicles.

The electric field is applied locally by plate electrodes, wrapped into a spongy cloth saturated with an aqueous solution of the selected drug. Iontophoresis is generally employed as an alternative solution to mesotherapy which, on the contrary, consists in executing multiple microinjections of specific medicaments into the cellulitis mass. Often associated with iontophoresis is a treatment phase providing application, by means of said electrodes, of the so-called Koltz waves, which deeply penetrate into the muscles causing the spontaneous contraction of same and therefore performing a sort of passive exercise.

A device for carrying out these treatments can be of the kind disclosed by EP-A-0 128 103, wherein a plurality of small, well-localized electrodes are placed on the surface of a non-conductive garment, thus applying the current on the body in substantially punctiform areas.

Another typology of treatments against cellulitis. is represented by ultrasonic hydrolipoclasis taking advantage of particular needle-shaped probes applied to the skin surface to utilize the lipolitic effect of ultrasonic waves.

It is apparent that during some of the above treatments (electrolipolysis, mesotherapy, ultrasonic idrolipoclasis) microtraumas may occur due to penetration of needles into the skin, in particular in the case of sensitive skin with fragile and superficial capillaries.

In addition, in the known art, electrotherapeutic treatments must be carried out by medical staff, at qualified centres.

Since many sessions spaced in time are necessary in order to achieve good results, this brings about the inconvenience that the patient must each time go to the session place to be submitted to treatment, which results in a temporary interruption of his/her working activity.

On the other hand, application of these treatments on one's own is of difficult accomplishment, due to the understandable difficulties encountered by the patient in preparing and positioning the electric-field generating circuit, in particular in the case in which the electrodes consist of needles.

It is also to note that these sessions are generally expensive, so that the long-lasting therapy involves a very high total cost for the patient.

It is a general object of the present invention to obviate the above mentioned drawbacks by providing a device enabling a patient to be submitted to the application of endermic electrotherapeutic treatments without the intervention of medical staff and without any necessity to address to qualified centres.

It is a further object of the invention to provide a device enabling elimination of microtraumas and capillary breakings due to the needle penetration into the skin.

In view of the above objects, in accordance with the present invention, a device for carrying out endermic electrotherapeutic treatments has been devised which comprises a pulse-current generator and a garment of fabric adapted to be worn by a patient, characterized in that the garment is formed by a plurality of electrically conductive discrete portions for contact with the patient's skin, the conductive discrete portions being separated by insulating portions the conductive portions being made of portions of electrically conductive fabric provided with respective terminals connected to the current generator by conductors to distribute the current onto the skin.

It is apparent that a device in accordance with the invention, since it can be personally worn by the patient, enables treatments to be constant in time, offering improved results as compared with a series of individual application sessions, at a lower total cost. In addition, since cellulitis is a pathology that is likely to recur easily in time, the device of the invention can be reutilized on the occasion of subsequent therapeutic application cycles which the patient will be eager to execute, since all inconveniences and commitments connected with a treatment in a specialized centre are eliminated.

It is to note that the electrically conductive elements to be utilized for the conductive portion of the sheath garment being the object of the invention can be variously selected from those known per se. For example, fabrics in which the fibre is twisted with at least one conductive wire are known and they are used to make garments such as wollen underwear, shirts, etc. in order to attenuate potential differences taking place between the different parts of the human body, thereby creating a substantial equipotentiality.

Such an electrically conductive fabric can be used to form discrete portions of the garment being part of the device in accordance with the invention, that is to accomplish descrete electric-current application areas, intentionally creating between said areas potential differences to be utilized for therapeutic purposes.

In order to better explain the innovatory principles of the present invention and the advantages it offers over the known art, a possible embodiment applying said principles will be described hereinafter, by way of example, with the aid of the accompanying drawings, in which:
- Fig. 1 is an elevation front view of the device in accordance with the invention;
- Fig. 2 is an elevation side view of the device in Fig. 1 seen from the right;
- Fig. 3 is a sectional view taken along line III-III in Fig. 2;
- Fig. 4 is a graph showing an example of a waveform of the electric current that can be utilized by the device of the invention.

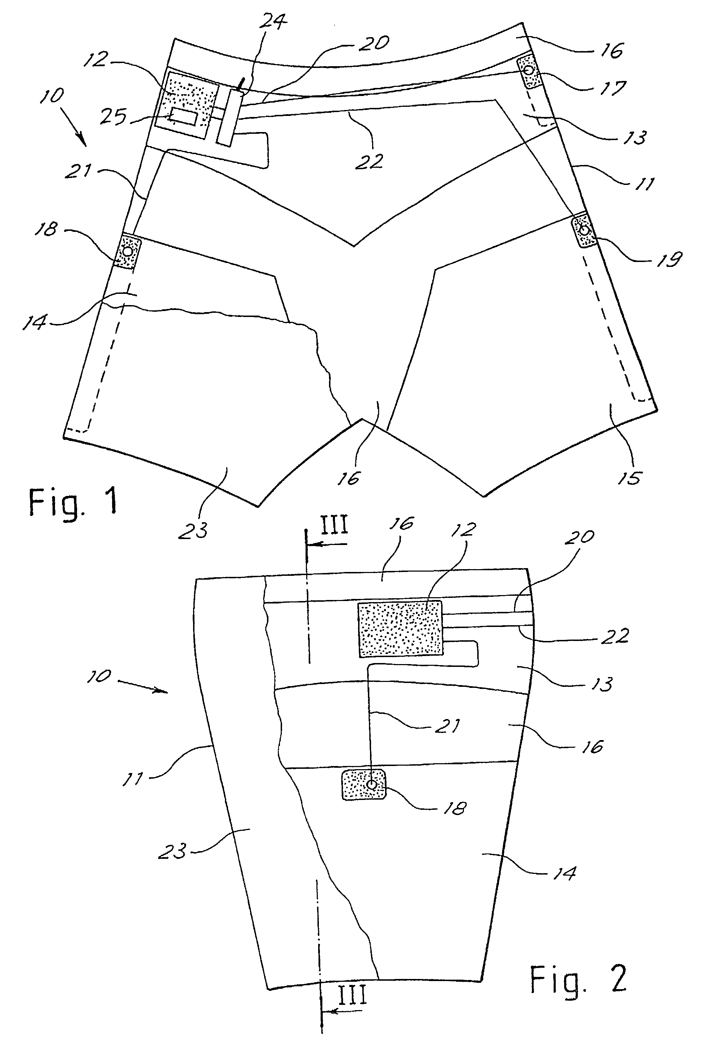

A device 10 for executing endermic electrotherapeutic treatments is shown in Figs. 1 and 2. It comprises an element briefly identified as a garment 11, adapted to be worn by a patient, and a current generator 12. In this embodiment, the garment 11 is shown in a form adapted to cover the patient's abdomen, glutei and thighs by elastically wrapping them, for direct treatments to these parts of the human body.

According to the innovatory principles of the present invention, the garment or sheath 11 consists of a plurality of discrete portions having electrically conductive and insulating features, respectively. With reference to the preferred embodiment of the device shown in the drawings, electrically conductive portions are denoted by 13, 14 and 15 and are disposed at the patient's waist (sheath belt) and thighs (sheath leggings) respectively, whereas insulating sheath portions are generally denoted by 16, and they separate the individual conductive portions from each other. In the example herein described three conductive poles or electrodes 13, 14 and 15 are shown. For particular requirements however, the sheath can be formed with a different number of conductive portions, disposed according to different configurations.

At the conductive portions there are electrical contact means or strips 17, 18, 19 provided with a sticky surface for steady application or sewn. The electrodes are connected to generator 12 by respective clips and conductor wires 20, 21, 22.

The insulating portions 16 can be made of a common non-conductive fabric, such as cotton. The conductive portions 13, 14, 15 on the contrary, are made to advantage of a cotton yarn consisting of textile fibres and metal wire. A yarn that has offered excellent results is that known under the trade name "Kind Star" (Registered Trademark).

The contact means 17, 18, 19 can extend along the conductive fabric (as shown in broken lines in Fig. 1) so as to ensure a good electric contact with the conductive fabric forming the electrodes. It is therefore ensured also a good equipotentiality of the surface of each electrode, thus avoiding electrical breaks due to seams, breakings or ladders of the conductive fabric.

The use of a conductive fabric is surely advantageous, also due to its structural likeness with the non-conductive fabric to which it is connected for obtaining the whole garment, so that the garment manufacture using machines of known type is facilitated.

However, a material having behavioural features similar to those of a fabric, even with a different structure can be also used.

A garment having the disclosed structure allows a good wearableness and does not hinder the normal sweating, and thus can be used for a long time.

A non-woven fabric or a like film having features of electrical conductivity can be utilised for the purpose, which fabric must be in any case connected to the adjacent non-conductive portions of the garment following the most appropriate technologies based on the constituent material, and therefore by sewing, gluing, welding and the like.

Obviously the conductive material, typically a metal material in any case incorporated in the garment wall, is required to have high features in terms of being inoxidizable and resisting acid corrosion, also taking into account the electrolytic effect of the carried currents and the environment where treatment is executed.

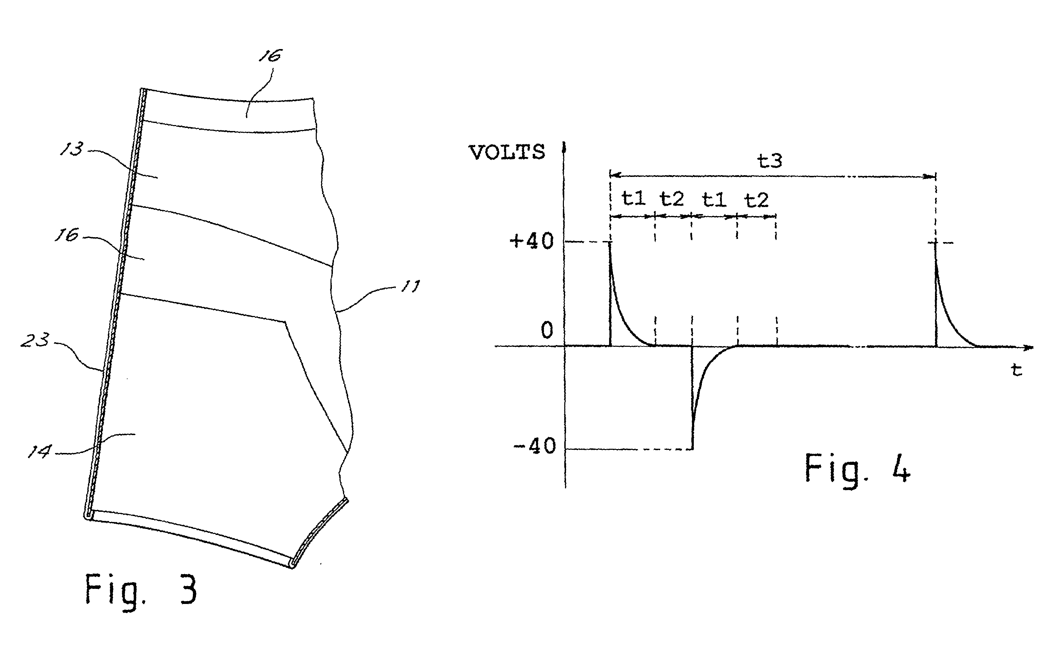

The conductive and insulating portions of the sheath can be externally covered with an insulating coating layer 23, for example consisting of a fabric of the type known under the trade name "Lycra" (Registered Trademark), to give the garment the required consistency and elasticity. The coating layer 23, only partly represented in Figs. 1 and 2, can be clearly seen in Fig. 3 where, for the sake of clarity, it has been shown without the section-indicating chain line. This coating layer promotes adhesion of the conductive fabric to the human body.

Generator 12 can be of reduced bulkiness (taking into account the weak currents involved), preferably of a portable type powered by an internal battery 25 or an AC/DC adapter. It can be of a known type in terms of circuits and adapted to generate electric currents characterized by waves known as "H waves", to create appropriate electric fields between the conductive portions of the sheath. Shown in Fig. 4 is a graph voltage-time of a current wave to be utilized with the device of the invention.

As can be viewed, the wave is formed of trains of alternating pulses having voltage output adjustable between ±20 ö ±80 volts, for example ±40 volts. The current output can be restricted at approximately 40 milliseconds.

With reference to the figure, experimental tests gave optimal characteristic values for time t1, t2, t3 corresponding to:
t1 = 1 millisecond;
t2 = 0.7-1 milliseconds;
t3 = 1000 milliseconds.

The initial edge of the pulses trains are spaced of t3 time. Each train consists of four positive/negative pulses. In that case, each train will last 15 milliseconds (with t2 = 1 millisecond).

A current with such a configuration has shown its capability of activating the blood and lymphatic microcirculations in a very satisfactory manner at the areas concerned with cellulitis pathologies, and of increasing the metabolic activity of the cells, resulting in a therapeutic action on cellulitis.

Generator 12 comprises a device 24 (e.g. a switch) for selection of the connections of the generator outputs to the three electrically conductive poles 13, 14, 15. In this way, different circuit configurations can be obtained and consequently different operating modes of the sheath.

For example, the generator can be connected between the two electrically conductive poles of the sheath leggings (connected in parallel to each other) and the waist pole.

As an alternative solution, the generator can be connected between the leggings poles, and the waist pole can be disconnected.

At this point it is apparent that the innovatory device of the invention enables achievement of the intended purposes.

Obviously, the above description of an embodiment applying the innovatory principles of the present invention is given for purposes of illustration only and is not to be interpreted as a limitation of the scope of the patent rights herein claimed.

For example, the sheath shape can be different than that herein shown and can vary depending on the body area concerned with treatment. In particular, it may comprise other portions adapted to completely cover the patient's legs, thus taking the form of tights.

In addition, it is to note that the sheath in accordance with the invention, by generating appropriate electric fields between predetermined descrete portions of the sheath, enables application of treatments typical of electrolipolysis for stimulating the blood circulation and the production of given body substances, without excluding the possibility of using it for iontophoresis treatments for introduction of drugs and active ingredients through the skin.

Also solved are the problems of the prior art connected with the difficulty in conveniently positioning the electrodes by unskilled persons.

An essential advantage of the invention is represented by the fact that the patient, by merely wearing the garment, is automatically able to position the electrode conductive areas in a correct manner. For the above reason, every possibility of a wrong positioning is excluded and the patient is not required to have any ability and specific preparation, which qualities are on the contrary needed for positioning of free electrodes onto the human body, as provided by the known art.

The generator can be hung to the belt so as to allow an optimal freedom of movement.

## Claims

1. A device for carrying out endermic therapeutic treatments, comprising a pulse-current generator (12) and a garment of fabric (11) adapted to be worn by a patient, **characterized in that** the garment (11) is formed by a plurality of electrically conductive discrete portions (13, 14, 15) for contact with the patient's skin, the conductive discrete portions being separated by insulating portions (16), the conductive portions (13, 14, 15) being made of portions of electrically conductive fabric provided with respective terminals (17, 18, 19) connected to the generator (12) by conductors (20, 21, 22) to distribute the current onto the skin.

2. A device according to claim 1, **characterized in that** the electrically conductive portions (13, 14, 15) are made of a cotton yarn fabric consisting of textile fibres and metal wire.

3. A device according to claim 1, **characterized in that** the discrete electrically conductive portions consist of a first conductive portion (13), or sheath belt disposed at the patient's waist, and second (14) and third (15) conductive portions or sheath leggings, disposed at the patient's thighs.

4. A device according to claim 1, **characterized in that** the conductive portions (13, 14, 15) and insulating portions (16) are externally covered with an insulating coating layer (23) giving the garment (11) the required mechanical features, in particular elasticity.

5. A device according to claim 1, **characterized in that** the current generator (12) is adapted to generate electric currents with H waves, to create electric fields between the conductive portions (13, 14, 15) of the sheath.

6. A device according to claim 5, **characterized in that** the H wave is formed of trains of alternating pulses separated by 15 milliseconds and having maximum voltage values of ±80 volts and a duration of 1 millisecond each pulse, each train having four positive/negative pulses.

7. A device according to claim 3, **characterized in that** the current generator (12) comprises a device for selection of the outputs to the electrically conductive poles (13, 14, 15) so as to connect alternately the generator between the leggings or between the leggings and the belt.

## Patentansprüche

1. Vorrichtung zum Durchführen endermatischer therapeutischer Behandlungen, welche einen Impulsstromgenerator (12) und ein Kleidungsstück aus einem Gewebe (11) aufweist, das für das Tragen durch einen Patienten angepaßt ist, **dadurch gekennzeichnet, daß** das Kleidungsstück (11) durch eine Vielzahl von elektrisch leitenden diskreten Abschnitten (13, 14, 15) für einen Kontakt mit der Haut des Patienten ausgebildet ist, wobei die leitenden diskreten Abschnitte durch isolierende Abschnitte (16) getrennt sind, und die leitenden Abschnitte (13, 14, 15), die aus Abschnitten eines elektrisch leitenden Gewebes hergestellt sind, mit entsprechenden Anschlüssen (17, 18, 19) versehen sind, die mit dem Generator (12) durch Leiter (20, 21, 22) verbunden sind, um den Strom auf der Haut zu verteilen.

2. Vorrichtung nach Anspruch 1, **dadurch gekennzeichnet, daß** die elektrisch leitenden Abschnitte (13, 14, 15) aus einem Baumwollgarngewebe bestehen, das aus textilen Fasern und Metalldraht besteht.

3. Vorrichtung nach Anspruch 1, **dadurch gekennzeichnet, daß** die diskreten elektrisch leitenden Abschnitte aus einem ersten leitenden Abschnitt (13) oder an der Hüfte des Patienten angeordneten Ummantelungsgürtel und zweiten (14) und dritten (15) an den Oberschenkeln des Patienten angeordneten leitenden Abschnitten oder Ummantelungsschenkelelementen besteht.

4. Vorrichtung nach Anspruch 1, **dadurch gekennzeichnet, daß** die leitenden Abschnitte (13, 14, 15) und die isolierenden Abschnitte (16) extern durch eine isolierende Beschichtungslage (23) abgedeckt sind, welche dem Kleidungsstück (11) die erforderlichen mechanischen Merkmale, insbesondere Elastizität, verleiht.

5. Vorrichtung nach Anspruch 1, **dadurch gekennzeichnet, daß** der Stromgenerator (12) für die Erzeugung von elektrischen Strömen mit H-Wellen angepaßt ist, um elektrische Felder zwischen den leitenden Abschnitten (13, 14, 15) der Ummantelung zu erzeugen.

6. Vorrichtung nach Anspruch 5, **dadurch gekennzeichnet, daß** die H-Welle aus Zügen abwechselnder Impulse getrennt durch 15 ms und mit maximalen Spannungswerten von ±80 Volt und einer Dauer von 1 ms für jeden Impuls gebildet wird, wobei jeder Impulszug vier positive/negative Impulse besitzt.

7. Vorrichtung nach Anspruch 3, **dadurch gekennzeichnet, daß** der Stromgenerator (12) eine Vorrichtung für die Auswahl der Ausgangssignale zu den elektrisch leitenden Polen (13, 14, 15) aufweist, um so abwechselnd den Generator zwischen die Schenkelelemente oder zwischen die Schenkelelemente und den Gürtel zu schalten.

## Revendications

1. Appareil de mise en oeuvre de traitements thérapeutiques endermiques, comprenant un générateur (12) d'impulsions de courant et un vêtement en tissu (11) conçu pour être porté par un patient, **caractérisé en ce que** le vêtement (11) est constitué d'une pluralité de parties discrètes conductrices d'électricité (13, 14, 15) destinées à entrer en contact avec la peau du patient, les parties discrètes conductrices étant séparées par des parties isolantes (16), et les parties conductrices (13, 14, 15) étant composées de parties de tissu conducteur d'électricité munies de bornes respectives (17, 18, 19) connectées au générateur (12) par des conducteurs (20, 21, 22) afin de distribuer le courant sur la peau.

2. Appareil selon la revendication 1, **caractérisé en ce que** les parties conductrices d'électricité (13, 14, 15) sont composées d'un tissu en fil de coton consistant en fibres textiles et en fil métallique.

3. Appareil selon de revendication 1, **caractérisé en ce que** les parties discrètes conductrices d'électricité consistent en une première partie conductrice (13), ou une ceinture-gaine disposée autour de la taille du patient, et en une deuxième (14) et une troisième (15) parties conductrices ou jambières-gaines disposées autour des cuisses du patient.

4. Appareil selon la revendication 1, **caractérisé en ce que** les parties conductrices d'électricité (13, 14, 15) et les parties isolantes (16) sont recouvertes, extérieurement, d'une couche d'enduction isolante (23) qui donne au vêtement (11) les caractéristiques mécaniques requises, en particulier son élasticité.

5. Appareil selon la revendication 1, **caractérisé en ce que** le générateur de courant (12) est conçu pour générer des courants électriques à ondes H, afin de créer des champs électriques entre les parties conductrices d'électricité (13, 14, 15) de la gaine.

6. Appareil selon la revendication 5, **caractérisé en ce que** les ondes H sont constituées de trains d'impulsions alternatives séparées de 15 millisecondes, et ayant des valeurs maximales de tension de ± 80 volts avec une durée de 1 milliseconde pour chaque impulsion, chaque train ayant quatre impulsions positives/négatives.

7. Appareil selon la revendication 3, **caractérisé en ce que** le générateur de courant (12) comprend un appareil permettant de sélectionner les sorties des portions conductrices d'électricité (13, 14, 15) de façon à connecter, alternativement, le générateur entre les jambières ou entre les jambières et la ceinture.
